# EUROPEAN PATENT SPECIFICATION

(11) **EP 4 178 595 B1**
(45) Date of publication and mention of the grant of the patent: **29.04.2026**
(21) Application number: 21733152.9
(22) Date of filing: 23.06.2021
(51) Int. Cl.: A61K 35/618, A61K 38/08, A61K 38/10, A61P 17/00, A61K 9/00, A61K 9/06, A61K 9/08, A61K 9/10, A61K 9/107, A61K 9/12, A61K 9/127, A61K 9/16, A61K 38/04, A61K 9/51, A61K 38/17

(54) **COMPOSITION COMPRISING A MELANOMA INHIBITORY ACTIVITY PROTEIN (MIA) INHIBITOR PEPTIDE AND SNAIL SECRETION, AND ITS USE IN THE TREATMENT OF VITILIGO**
ZUSAMMENSETZUNG, BESTEHEND AUS EINEM MELANOM-INHIBIERENDEN AKTIVITÄTSPROTEIN (MIA)-INHIBITORPEPTID UND SCHNECKENSEKRET, UND DEREN VERWENDUNG ZUR BEHANDLUNG VON VITILIGO
COMPOSITION COMPRENANT UN PEPTIDE INHIBITEUR DE LA PROTÉINE INHIBITRICE DE L'ACTIVITÉ DU MÉLANOME (MIA) ET DE LA SÉCRÉTION D'ESCARGOT, ET SON UTILISATION DANS LE TRAITEMENT DU VITILIGO

(30) Priority: 09.07.2020 EP 20382617
(43) Date of publication of application: 17.05.2023
(73) Proprietor: Bella Aurora Labs, S.A., 08690 Santa Coloma de Cervelló (Barcelona) (ES); Bordignon, Matteo, 35126 Padova (IT)
(72) Inventor: HERNÁNDEZ NAVARRO, Sergi, 08690 Santa Coloma de Cervelló (BARCELONA) (ES); SEGURA TEJEDOR, Jordi, 08690 Santa Coloma de Cervelló (BARCELONA) (ES); BORDIGNON, Matteo, 35126 Padova (IT)
(74) Representative: Pons IP
(86) International application number: PCT/EP2021/067203
(87) International publication number: WO 2022/008251

(56) References cited:
- WO-A1-2019/185696
- WO-A2-2015/075116
- US-A1- 2014 004 130
- RAFFAELLA COSENTINO: "News Vitiligine: cosa fare dopo l'estate e una nuova opportunità terapeutica", PENSALLASALUTE -GIORNALE ONLINE, WEB TV, 24 June 2020 (2020-06-24), XP055756012, Retrieved from the Internet <URL:https://pensallasalute.com/cura-definitiva-per-la-vitiligine/> [retrieved on 20201202]
- SHAB ARNA SHAB ET AL: "Der Hals lügt nicht - Tixel die sichere und risikoarme Therapieoption zur Behandlung von Falten im Bereich des Halses: Fallbericht", KOSMETISCHE MEDIZIN, 2 June 2020 (2020-06-02), pages 26 - 29, XP055758397, Retrieved from the Internet <URL:https://www.med-aesthet.de/files/medaesthet/aktuelles/artikel-sanfte-halsstraffung-mit-tixel-kosmetische-medizin-02-2020.pdf> [retrieved on 20201209]
- ROSELYNE Y. WAGNER ET AL: "Altered E-Cadherin Levels and Distribution in Melanocytes Precede Clinical Manifestations of Vitiligo", JOURNAL OF INVESTIGATIVE DERMATOLOGY, vol. 135, no. 7, 1 July 2015 (2015-07-01), NL, pages 1810 - 1819, XP055370517, ISSN: 0022-202X, DOI: 10.1038/jid.2015.25

## Description

### Technical field

The present invention relates to a pharmaceutical composition comprising a melanoma inhibitory activity protein (MIA) inhibitor peptide, which has sequence SEQ ID NOs: 40, or is a derivative thereof, snail secretion and at least one pharmaceutically acceptable excipient; for the treatment and/or prevention of vitiligo.

### State of the art

Vitiligo is an acquired skin disorder characterized by hypopigmented or depigmented areas and has a prevalence of about 0.5 to 2% in the world population.

Non-segmental vitiligo (NSV) is the most common clinical form of the disease, wherein the patches of depigmentation are usually bilateral and with some form of symmetry.

Vitiligo most commonly involves the face, digits, dorsal hands, flexor wrists, elbows, knees, shins, dorsal ankles, armpits, inguinal area, anogenital area, umbilicus, and nipples.

A loss of epidermal melanocytes is the pathologic hallmark of vitiligo, but the aetiology of the disease is multifactorial and still poorly understood. For example, among the factors which can contribute to non-segmental vitiligo are immunologic factors, oxidative stress, or a sympathetic neurogenic disturbance. On the other hand, it is thought that there is also some genetic predisposition to this disease (Taïeb et al., Vitiligo, N. Engl. J. Med., 2009, 360, 160-169).

Among the pharmacological treatments proposed so far are, for example, topical corticosteroids, topical macrolide immunomodulators, namely, the calcineurin inhibitors tacrolimus and pimecrolimus, and vitamin D3 analogues and antioxidants. On the other hand, the most used non-pharmacological treatment are ultraviolet radiations, both in the range of UVA and UVB (Guerra et al., Vitiligo: pathogenetic hypotheses and targets for current therapies, Current Drug Metabolism, 2010, 11, 451-467).

These available treatments are barely effective and some of them may involve some risk of adverse effects.

More recently, it has also been suggested the involvement of the protein MIA ("*Melanoma inhibitory activity*") in the pathogenesis of non-segmental vitiligo based on the finding that, unlike in normal melanocytes, the MIA protein is expressed in vitiliginous skin and may cause the detachment of melanocytes (melanocytorrhagy) due to its interaction with alpha5beta1 integrin, thus perturbing the normal attachment of melanocytes to basal membrane (Bordignon et al., Role of alpha5beta1 integrin and MIA (melanoma inhibitory activity) in the pathogenesis of vitiligo, J. Dermatol. Sci., 2013, 71(2), 142-5). In an *in-vivo* mice model, it was confirmed that the injection of MIA in the tail of the animals induced vitiligo-like depigmentation in the injected area (Bordignon et al., MIA (melanoma inhibitory activity) is able to induce vitiligo in an in-vivo mice model by direct injection in the tail, Vitiligo International Symposium 2016, Meeting Abstracts).

Therefore, MIA is suggested as target for vitiligo therapy, and in the international patent application WO-A-2012/127352 a number of peptides and peptide derivatives which are MIA inhibitors are proposed for the prevention and treatment of vitiligo, by a mechanism involving the binding of the inhibitors to MIA and thus avoiding the interaction of MIA with alpha5beta1 integrins, which is the cause of melanocytorrhagy.

It is noticeable, however, that the document WO-A-2012/127352 does not provide any working example showing the effectiveness of those substances for vitiligo therapy and, actually, it does not give any hint about suitable methods or compositions for using those MIA inhibitors for effectively treating vitiligo.

The document US2014/004130 A1 discloses peptides, modified peptides and antibody or antibody fragment, which inhibit the activity of MIA (Melanoma Inhibitory Activity), for detecting, preventing and curing vitiligo, wherein said MIA inhibitor peptides comprises the sequences SEQ ID Nos: 1 to 49 disclosed in the present application.

The document *Raffaella Cosentino, News Vitiligine: cosa tare dopo l'estate e una nuova opportunita terapeutica", Pensallasalute -giornale online, Web TV, 24 June* 2020 discloses the functioning of the MIA protein, the protein that causes vitiligo, and the preservation of the adhesiveness of melanocytes. Regarding vitiligo, snail slime proteins favor the adhesion of melanocytes by stimulating the production of adhesion molecules by the skin. The document *Shab Arna Shab et al, Der Hals lügt nicht - Tixel die sichere und risikoarme Therapieoption zur Behandlung van Falten im Bereich des Halses: Fallbericht", Kosmetische Medizin, 2 June 2020* also discloses the use of snail secretion in the treatment of vitiligo.

The document WO2015/075116A2 discloses targeted microcapsules or nanocapsules comprising a skin whitening compound to target melanocytes with a peptide, which is a MC1 receptor agonist, and their use for the cosmetic prevention and/or cosmetic treatment of cutaneous dark spots.

The document WO 2019/185696 A1 palmitoyl pentapeptide-4 conjugated gold nanoparticles, compositions comprising them, wherein said peptides improve stability and good penetration, and use thereof in cosmetic treatments, as well as methods for obtaining said nanoparticles.

The document Roselyne Y. Wagner et al, Altered E-Cadherin Levels and Distribution in Melanocytes Precede Clinical Manifestations of Vitiligo, Journal of investigative dermatology, vol. 135. no. 7. 1 July 2015 discloses that E-cadherin (Ecad), which mediates the adhesion between melanocytes and keratinocytes in the epidermis, is absent from or discontinuously distributed across melanocyte membranes of vitiligo patients long before clinical lesions appear and that Ecad is required for melanocyte adhesiveness to the basal layer under oxidative and mechanical stress, establishing a link between silent/preclinical, cell-autonomous defects in vitiligo melanocytes and known environmental stressors accelerating disease expression.

Therefore, there is still the need to provide effective and safe compositions for the treatment and prevention of vitiligo.

### Object of the invention

The object of the present invention is a pharmaceutical composition comprising a MIA inhibitor peptide which has sequence SEQ ID NO: 40, snail secretion and at least one pharmaceutically acceptable excipient.

Another aspect of the present invention is a kit comprising the pharmaceutical composition of the invention and a dietary supplement comprising an anti-oxidant.

Another aspect of the invention is such composition or said kit for use in the prevention and/or treatment of vitiligo.

### Description of the Drawings

Figure 1 shows three illustrative examples for testing the efficacy of three different compositions in a single patient, for comparative purposes.
Figure 2 shows a picture of one of the patients enrolled in the trial described in Example 4, before and after the treatment, wherein the left side of the face was treated with the composition A and the right side of the face was treated with the composition B.
Figure 3 shows a picture of one of the patients enrolled in the trial described in Example 4, before and after the treatment, who was treated with the composition C.

### Detailed description

The present invention is defined in the appended claims. The object of the present invention is a pharmaceutical composition comprising:
(a) a MIA inhibitor peptide which has sequence SEQ ID NO: 40, or a derivative thereof, wherein derivative means that the peptide is acetylated at the N-terminus and/or amidated at the C-terminus;
(b) snail secretion; and
(c) at least one pharmaceutically acceptable excipient.

The authors of the present invention as claimed have developed a pharmaceutical composition comprising the combination of a MIA inhibitor peptide and snail secretion that, surprisingly, unlike the treatments available so far in the state of the art, is outstandingly effective for treating vitiligo.

Along the present description, as well as in the claims, the singular expressions, generally preceded by the articles "a", "an" or "the", are meant to include also the plural forms, unless the context clearly indicates otherwise. All percentages are expressed by weight, unless specifically stated otherwise. Numeric values preceded by the term "about" are meant to include also a certain variation around such value, namely a variation or ±5% of the stated amount. Numeric ranges defined by lower and upper endpoints are meant to include also said stated endpoints and they also include any narrower sub-range.

The amino acids are represented herein using the one-letter code, as is well-known by the skilled in the art, according to IUPAC-IUB Commission on Biochemical Nomenclature.

### MIA inhibitor peptide

MIA inhibitor peptide is a substance having activity as inhibitor of the human melanoma inhibitory activity (MIA) protein, in particular, those disclosed in the international patent application WO-A-2012/127352, namely, a peptide having a sequence selected from SEQ ID NO: 1 to SEQ ID NO: 49:
- SEQ ID NO: 01 VPHIPPN
- SEQ ID NO: 02 MPPTQVS
- SEQ ID NO: 03 QMHPWPP
- SEQ ID NO: 04 QPPFWQF
- SEQ ID NO: 05 TPPQGLA
- SEQ ID NO: 06 IPPYNTL
- SEQ ID NO: 07 AVRPAPL
- SEQ ID NO: 08 GAKPHPQ
- SEQ ID NO: 09 QQLSPLP
- SEQ ID NO: 10 GPPPSPV
- SEQ ID NO: 11 LPLTPLP
- SEQ ID NO: 12 QLNVNHQARADQ
- SEQ ID NO: 13 TSASTRPELHYP
- SEQ ID NO: 14 TFLPHQMHPWPP
- SEQ ID NO: 15 VPHIPPNSMALT
- SEQ ID NO: 16 RLTLLVLIMPAP
- SEQ ID NO: 17 YNLPKVSSNLSP
- SEQ ID NO: 18 MPPTQVSKFRLI
- SEQ ID NO: 19 ANIDATPLFLRA
- SEQ ID NO: 20 LLRTTETLPMFL
- SEQ ID NO: 21 SALEPLV
- SEQ ID NO: 22 GSPTPNA
- SEQ ID NO: 23 APSHATH
- SEQ ID NO: 24 TTVGHSD
- SEQ ID NO: 25 THFSTFT
- SEQ ID NO: 26 SLLLDTS
- SEQ ID NO: 27 SVAMKAHKPLLP
- SEQ ID NO: 28 NTIPGFASKSLD
- SEQ ID NO: 29 VSNYKFYSTTSS
- SEQ ID NO: 30 VSRHQSWHPHDL
- SEQ ID NO: 31 HLNILSTLWKYR
- SEQ ID NO: 32 HNASPSWGSPVM
- SEQ ID NO: 33 SHPWNAQRELSV
- SEQ ID NO: 34 HHWPFWRTLPLS
- SEQ ID NO: 35 WHTKFLPRYLPS
- SEQ ID NO: 36 NNTSFTVVPSVP
- SEQ ID NO: 37 SHLSTWKWWQNR
- SEQ ID NO: 38 FHWHPRLWPLPS
- SEQ ID NO: 39 WHWTYGWRPPAM
- SEQ ID NO: 40 FHWRYPLPLPGQ
- SEQ ID NO: 41 WHWPLFIPNTTA
- SEQ ID NO: 42 WHNGIWWHYGVR
- SEQ ID NO: 43 HHLNYLWPWTRV
- SEQ ID NO: 44 FWHRWSTFPEQP
- SEQ ID NO: 45 WHMSYFWTRPPQ
- SEQ ID NO: 46 FHLNWPSRADYL
- SEQ ID NO: 47 WHKNTNWPWRTL
- SEQ ID NO: 48 ALSPSQSHPVRS
- SEQ ID NO: 49 GTQSTAIPAPTD

In the composition of the invention, the MIA inhibitor peptide has the sequence SEQ ID NO 40.

A derivative of a peptide having the sequence SEQ ID NO: 40 means a peptide with some small variations around these sequences, still retaining the MIA inhibitory activity.

The MIA inhibitor peptide derivative is a peptide which is acetylated at the N-terminus and/or amidated at the C-terminus, preferably is acetylated at the N-terminus and amidated at the C-terminus.

The MIA inhibitor is a peptide of SEQ ID NO: 40; and wherein the sequence is acetylated at the N-terminus and/or amidated at the C-terminus, and preferably is acetylated at the N-terminus and amidated at the C-terminus.

The "MIA inhibitor peptide" or "MIA inhibitor" terms, as used interchangeably herein, refer widely to the MIA inhibitor substance used in the composition, either if it is strictly a peptide sequence or a modified sequence as disclosed above.

In one embodiment, the MIA inhibitor peptide has a sequence as defined above and is not modified.

The peptide and derivative thereof can be prepared following standard methods in peptide synthesis, typically, by solid phase synthesis, or may be obtained commercially from several sources.

The composition of the invention as claimed may comprise one or more MIA inhibitor peptide, i.e. a single MIA inhibitor substance or the combination of two or more MIA inhibitors. When more than one MIA inhibitor is used, they may be used in any proportion and, therefore, the amount of MIA inhibitor as stated herein means the total amount of MIA inhibitJor substances contained in the composition.

The activity as MIA inhibitors of the peptides of sequences 1 to 49 was determined in a phage display screening assay using recombinant human MIA.

The derivatives of the peptides of sequences 1 to 49, as discussed above, can be tested for their MIA inhibitory activity using standard methods, for example, a ligand binding assay using recombinant human MIA.

The MIA inhibitor peptide can be added as such to the composition or may be encapsulated within different polymeric materials or conjugated to different carrier substances for improving its transdermal delivery through the stratum corneum to reach the target MIA protein in the vitiliginous melanocytes, which are located in the bottom layer of the epidermis, the stratum basale, attached to the basement membrane.

Encapsulation techniques are a well-known in the art for the delivery of active ingredients.

In one embodiment, the MIA inhibitor peptide is micro-encapsulated using liposome technology.

Liposomes are commonly used in dermal formulations for improving penetration of actives through the skin layer. As is well known in the art, liposomes are spherical vesicles with sizes generally in the range between 60 nm and 300 nm and are most often composed of phospholipids which form at least one phospholipid bilayer, but may also include other lipids. Liposomes contain hydrophilic cores in which hydrophilic actives may be encapsulated, while hydrophobic actives are incorporated in the bilayer, so liposomes are suitable carriers for both hydrophilic and lipophilic actives. Liposomes may be prepared by well-known techniques; in general, the preparation methods involve mixing the membrane-forming lipids, in an organic phase, drying, subsequent hydration of the lipids and further size reduction by different mechanical treatment such as sonication, extrusion or homogenization.

In another embodiment, the MIA inhibitor is encapsulated within a microcapsule or nanocapsule made of a biodegradable polymer, as are well-known in the art, for example, including polyesters, polyamino acids, polyalkyl cyanoacrylates, polyphosphazenes, or polyethylene oxide. Common polymers used for encapsulation are, for example, polyvinyl alcohol (PVA), poly(lactic acid) (PLA), poly(lactic-co-glycolic acid) (PLGA) or their block co-polymers with poly(ethylene oxide) or poly(ethylene glycol). Other suitable biodegradable polymers are poly(propylene fumarate-co-ethylene glycol) [P(PF-co-EG)] block copolymer, poly-anhydride poly(fumaric-co-sebacic) anhydride, alginate, dextran, chitosan, hydroxyapatite, collagen, fibrin, hyaluronic acid, carbomers, and mixtures thereof.

In a particular embodiment, the MIA inhibitor is encapsulated within a microcapsule or nanocapsule which has attached on the outer surface a specific peptide which is a melanocortin 1 (MC1) receptor agonist and those microcapsules or nanocapsules are designed for the targeted delivery of the encapsulated MIA inhibitor to melanocytes.

These targeted microcapsules or nanocapsules have typically a size distribution from 10 nm to 10000 nm, preferably from 50 nm to 5000 nm, more preferably from 100 nm to 1000 nm, still more preferably from 150 nm to 450 nm, and still more preferably from 180 nm to 400 nm, as disclosed in WO-A-2015/075116 op. *cit.* The size of the microcapsules may be determined by Scanning Electron Microscopy (SEM).

The microcapsules are preferably polymeric, generally made of one or more biodegradable polymers, for example, as those disclosed above. It is required that at least one of the polymers forming the targeted microcapsule bears carboxylic groups for binding the peptide the MC1 receptor agonist peptide to the outer surface, by coupling said carboxylic groups with the amine terminal groups in the peptide.

The microcapsules may optionally be bilayered polymeric microcapsules comprising a core polymer (or "inner layer polymer"), and an outer shell polymer (or "outer layer polymer"). For example, the core polymer may be poly (D,L-lactide-co-glycolide) (PLGA) and the outer shell polymer may be polyvinyl alcohol (PVA).

The preparation of said targeted microcapsules or nanocapsules involves the mixture of the active ingredient (MIA inhibitor peptide in this case) and the polymer(s) forming the capsule in a suitable solvent, and a further step of coupling the MC1 receptor agonist peptide, which confers affinity towards melanosomes, to the outer surface of the microcapsule or nanocapsule. This step can be performed before or after forming the capsule, preferably after forming the capsule.

The MC1 receptor agonist coupled to the outer surface of the microcapsule or nanocapsule is a peptide of the formula:

R₂-Ser-Tyr-Ser-Nle-Glu-His-DPhe-Arg-(AA)-Gly-Lys-DPro-Val-R₁

or a pharmaceutically acceptable salt or solvate thereof, wherein:
- R₁ is the radical -NH-(CH₂)₃-O-(CH₂CH₂O)ₙ-(CH₂)₃-NH₂, wherein n is an integer from 1 to 10; for example, n is 1 and R₁ derives from ethylene glycol bis (3-aminoproyl) ether (CAS No. 2997-01-5); in another example, n is 2 and R₁ derives from diethylene glycol bis (3-aminoproyl) ether, also called 4,7,10-trioxa-1,13-tridecanediamine (CAS No. 4246-51-9);
- R₂ is selected from (C₁₋₂₄ alkyl)-CO-, (C₂₋₂₄ alkenyl)-CO- and (C₆₋₁₀ aryl)-CO-; for example, R₂ is selected from acetyl, propanoyl, pentadecanoyl, hexadecanoyl and heptadecanoyl (palmitoyl); and
- AA is an amino acid containing an aromatic group; for example, AA may be selected from tryptophan, 3-(2-naphthyl)-D-alanine, 3-amino-3-(1-naphthyl)-propionic acid, 3-amino-3-(biphenyl)-propionic acid, phenylalanine, tyrosine, histidine, 5-hydroxytryptophan and L-3,4-dihydroxy-phenylalanine.

In another embodiment, the MIA inhibitor peptide is conjugated with nanoparticles, namely, with lipid nanoparticles, polymer nanoparticles, magnetic nanoparticles or metallic nanoparticles, which may act as carriers to facilitate the penetration of the MIA inhibitor through the stratum corneum.

In one particular embodiment, the MIA inhibitor peptide is conjugated with gold nanoparticles, which are described to improve penetration through skin. Those nanoparticles have a gold core coated with the MIA inhibitor peptide. The MIA inhibitor peptide is conjugated to the gold nanoparticles, typically by means of an electrostatic ionic bond between the negative charges of the gold surface of the nanoparticles and the positive charges of amino acids of the peptide carrying amino groups in the side chain (lysine, arginine, histidine, asparagine or glutamine). The gold nanoparticles have a size of less than 1000 nm, preferably comprised between 50 and 200 nm. The conjugates may be prepared by reducing Au (III) to Au (0) with a reducing agent and treating the gold nanoparticles obtained with the MIA inhibitor peptide.

The amount of the MIA inhibitor peptide in the composition of the invention, either if it is in free form or encapsulated or conjugated with a carrier, as disclosed above, is typically comprised between 0.0001% and 1%, preferably comprised between 0.0005% and 0.1%, and more preferably comprised between 0.001% and 0.05%, wherein the percentages are expressed as weight of the MIA inhibitor relative to the total weight of the composition.

### Snail secretion

Snail secretion, also known as snail slime, is a kind of mucus or bodily secretion which is produced by snails (gastropods), by glands located in the foot of the gastropod. This mucus is made of water and a complex mixture of substances, including proteoglycans, glycosaminoglycans, glycoprotein enzymes, hyaluronic acid, copper peptides, antimicrobial peptides, and metal ions; the main components are allantoin, collagen, elastin and glycolic acid.

This secretion may be collected and recovered from living snails. In general, after subjecting the gastropods to certain stress conditions, the secretion is collected, and filtered.

Snail secretion is generally in the form of a liquid, the pH is generally comprised in the range 5-8, the density is typically from 1.0 to 1.1 g/ml and the dry residue is generally comprised between 0.1% and 3%, preferably comprised between 0.2% and 2%, more preferably comprised between 0.25% and 1%, and still more preferably comprised between 0.3% and 0.7%. The protein content is typically in the range 0.1-3 mg/ml.

Snail secretion is commercially available from several sources, for example, from the company Cobiosa, Spain (sold as product Poly-Hexilan PF) or from the company CENTISIA Laboratorio di Fitocosmesi di Bruno Dott.ssa Laura Francesca & C. s.a.s., Italy (sold as Prodotto 580038), among others.

Different species of gastropods may be used to obtain this secretion, including, among others, *Achatina fulica, Cornu aspersum* (or *Helix aspersa* or *Cryptomphalus aspersa or Cantareus aspersus), Helix pomatia, Helix hortensis, Helix nemoralis, Helix cardidula, Helix tchthyomma, Helix fructicicola, Helix strigella, Helix fruticum, Helix bidens, Helix arbostorum, Helix rotundata, Helix aculeata, Helix pulchella, Helix personata, Helix holoserica, Helix aperta, Helix parnassia, Helix alonensis, Helix candidissima, Helix pisana,* or *Helix gualteviana.* In a particular embodiment, the snail secretion is obtained from *Cornu aspersum,* also known as *Helix aspersa, Cryptomphalus aspersa* or *Cantareus aspersus.*

The medicinal and cosmetic use of snail secretion has been disclosed in the state of the art, for example, as antimicrobial agent, for wound repair, for skin protection, or as anti-aging agent, among other uses.

The authors of the present invention as claimed have surprisingly discovered that the combination of a MIA inhibitor substance and snail secretion provides outstanding results in the treatment of vitiligo.

Indeed, as disclosed in the comparative Example 3, it was found that while a composition comprising snail secretion alone did not show any effect on vitiligo depigmented skin, the combination of this substance with MIA inhibitors provided the best anti-vitiligo effect, with notable re-pigmentation of the affected areas.

The amount of snail secretion in the composition of the invention as claimed is typically comprised between 0.5% and 15%, preferably comprised between 1% and 10%, more preferably comprised between 2% and 8% and still more preferably is 5%, wherein the percentages are expressed as weight of the snail secretion relative to the total weight of the composition.

### Compositions of the disclosure

The composition of the invention as claimed comprises a MIA inhibitor peptide which has sequence SEQ ID NO: 40, or is a derivative thereof, wherein derivative means that the peptide is acetylated at the N-terminus and/or amidated at the C-terminus; snail secretion and at least one pharmaceutically acceptable excipient.

The term "excipient" or "pharmaceutically acceptable excipient" means a component of a pharmaceutical composition that is not an active ingredient, which is useful for preparing the pharmaceutical composition and is generally safe and non-toxic for human pharmaceutical use. In particular, the composition of the disclosure is typically intended to be used externally, topically applied over the affected skin area, so the pharmaceutically acceptable excipient is specifically "dermatologically acceptable", i.e., it is suitable and non-toxic for use in contact with human skin tissue.

In one embodiment, the composition according to present invention as claimed is for topical administration, i.e., it is to be spread over the skin in the area to be treated, namely, in the area showing vitiliginous depigmentation or prone to show vitiliginous depigmentation. Topical, dermal, or cutaneous administration are used herein interchangeably.

Any type of formulation suitable for topical administration may be used. Typically, the composition of the disclosure is in the form of cream, gel, cremigel, lotion, paste, foam, solution, suspension, emulsion, milk, or stick preparation, for example, which are well-known in the art. A cremigel, for example, is a type of formulation halfway between a cream and a gel: unlike a cream, a cremigel is a gelled aqueous solution or a gelled oil, but it looks opaque as a cream, usually white in colour.

Suitable carriers may be, for example, anhydrous, as mixtures of fats, waxes, animal and plant oils and solid and liquid hydrocarbons. Or the carrier may be water or an aqueous solution of hydrophilic substances. Suitably, the carrier may be in the form of an emulsion. Emulsions may be, typically, oil-in-water emulsions, water-in-oil emulsions, water-in-oil-in-water, oil-in-water-in-oil or water-in-silicone emulsions. An emulsion may generally be described as having a continuous aqueous phase (oil-in-water and water-in-oil-in-water) or a continuous oil phase (water-in-oil and oil-in-water-in-oil). The oil phase may comprise silicone oils, non-silicone oils such as paraffin hydrocarbons, fatty alcohols (e.g. stearyl alcohol, cetyl alcohol or cetostearyl alcohol), fatty acids (e.g. stearic acid, oleic acid), fatty acid esters (e.g. isopropyl myristate, isopropyl palmitate), waxes or plant oils (e.g. castor oil, canola oil, cottonseed oil, jojoba oil or arachis oil), or mixtures thereof. The aqueous phase may comprise water or a water solution of hydrophilic substances, such as alcohols (e.g. ethanol, isopropyl alcohol), polyols (e.g. glycerol, sorbitol), alpha hydroxy acids, amino acids, protein hydrolysates, simple sugars, or polysaccharides.

Emulsifiers, which are common components of emulsions, are surface-active agents (surfactants) and include non-ionic surfactants, anionic surfactants, cationic surfactants and amphoteric surfactants. Non-ionic surfactants include, among others, ethoxylated fatty alcohols, ethoxylated fatty acid esters, alkyl glucosides or alkyl oligoglucosides, ethoxylated sorbitan fatty acid esters, monoglycerol/polyglycerol fatty acid esters, ethoxylated glycerin monesters, ethoxylated polyglyceryl esters, alkyl dimethylamine oxides, or poloxamers, among others. Anionic surfactants include alkaline soaps, alkyl sulphates, alkyl ether sulphates, alkyl sulphosuccinates, alkyl phosphates, acyl sarcosinates or acyl isethionates, among others. Cationic surfactants include quaternary ammonium salts, or pyridine salts, among others. Amphoteric surfactants include imidazoline derivatives, betaines, amidobetaines and sulphobetaines.

Other common ingredients in the formulation are, for example, emollients, humectants, preservatives, viscosity controlling agents, antioxidants, pH regulators, UV filters, chelating agents, perfumes and colorants. Common emollients are, for example, paraffin hydrocarbons, silicones, fatty alcohols, fatty acids, esters of fatty acids with alcohols, triglycerides, ceramides, phospholipids and waxes. Humectants include polyhydroxy alcohols, proteins and hydroxyl acids. Common preservatives include sorbic acid and its salts, benzoic acid and its salts, parabens, imidazolidinyl urea, diazolidinyl urea, DMDM hydantoin, sodium hydroxymethylglycinate, methylchloroisothiazolinone/methylisothiazolinone, benzyl alcohol and 2-phenoxyethanol, among others. Other additives may also be added for controlling the viscosity of the formulation, for example, xanthan gum, gellan gum, acacia, carrageenans, chitosan, collagen, tragacanth, pectin, starch derivatives, carbomers, cellulose derivatives (hydroxyethylcellulose, hydroxypropylcellulose, hydroxypropyl methylcellulose, methylcellulose, ethylcellulose, or carboxymethylcellulose, for example), polyamides, glutamides, colloidal silica or waxes (e.g. beeswax or vegetable waxes), among others.

Some ingredients of the composition may act as penetration enhancers, to facilitate the penetration of the active ingredients throughout the stratum corneum. Common penetration enhancers are, for example, short chain alcohols such as ethanol or isopropyl alcohol; long chain alcohols such as decanol, hexanol, lauryl alcohol, myristyl alcohol, octanol, octyldodecanol or oleyl alcohol; cyclic amides such as azone; esters such as ethyl acetate octyl salicylate, padimate O, ethyl oleate, glyceryl monoleate, glyceryl monocaprate, glyceryl tricaprylate, isopropyl myristate, isopropyl palmitate, propylene glycol monolaurate, or propylene glycol monocaprylate; ether alcohols such as Transcutol^{®}; fatty acids such as lauric acid, linoleic acid, linolenic acid, myristic acid, oleic acid, palmitic acid, stearic acid or isostearic acid; glycols such as dipropylene glycol, propylene glycol, 1,2-butylene glycol or 1,3- butylene glycol; pyrrolidones such as *N*-methyl-2-pyrrolidone or 2-pyrrolidone; sulphoxides such as decylmethyl sulphoxide or dimethyl sulphoxide; anionic surfactants; cationic surfactants; non-ionic surfactants; terpenes such as eugenol D-limonene, menthol, menthone, farnesol or neridol.

The composition of the disclosure has typically a slightly acidic pH, close to the physiological pH of the skin. Common acidity regulators are organic acids, including hydroxy acids and fatty acids. Some of the most common pH regulators in cosmetic emulsions are hydroxy acids such as lactic or citric acid.

One excipient can perform more than one function. The above cited ingredients, as well as many others suitable dermatological formulation excipients, are well-known to the skilled formulator of topical compositions. Such ingredients are commercially available from several companies, such as Comercial Química Massó, SA, Evonik, DuPont or Dow Corning, among others.

The preparation of the topical composition is made according to procedures well-known to the skilled in the formulation of dermatological compositions, generally involving simple steps of mixing, and optionally heating the component ingredients.

A cream, for example, can be typically prepared by warming the oily phase and the aqueous phase separately to a temperature of 60 to 80° C, and then mixing under stirring to prepare the emulsion.

Typically, for example, a cream, gel, or lotion base formulation is first prepared, using standard ingredients and procedures well-known in the art, and then snail secretion and the MIA inhibitor peptide (either in free form or encapsulated) are added and thoroughly mixed, and the pH of the composition is finally adjusted.

### Use of the composition

As shown in the prospective clinical trials described in Examples 3 and 4, the composition of the invention as claimed is outstandingly effective for treating vitiligo, with apparent re-pigmentation of the treated depigmented areas.

Another aspect of the disclosure is, therefore, the composition of the invention as claimed for use in the prevention and/or treatment of vitiligo.

The treatment of vitiligo is understood as the procedure aimed to improve vitiligo symptoms, namely, to partially or totally restore the normal pigmentation of the depigmented or hypopigmented skin patches.

In one embodiment, the prevention and/or treatment of vitiligo relates to the prevention and/or treatment of non-segmental vitiligo.

The prevention of vitiligo is aimed to prevent the onset of vitiligo symptoms and is addressed to subjects which are known to be prone to suffer from vitiligo, either for having been early diagnosed, before the appearance of symptoms, for example, by detecting the presence of MIA protein in the melanocytes, or for having some known genetic predisposition to suffer from this disease. The prevention of vitiligo also includes the treatment of patients who had previously suffered from vitiligo and have recovered from the disease, i.e., who have achieved re-pigmentation of the vitiliginous depigmented skin, to prevent them from suffering again from vitiligo symptoms.

Typically, the composition of the disclosure is administered topically, i.e., it is spread over the vitiliginous skin patches.

In one embodiment, the composition of the disclosure may be delivered transdermally, for example, via iontophoresis, ultrasound or using microneedles.

The amount of the composition to be used, considered to be "therapeutically effective" can widely vary depending on many factors, for example, the severity and progression stage of the disease, or the specific formulation used. The amount can be easily adjusted in each case by the skilled practitioner. In general, the amount of the topical composition applied to the affected skin may range from 0.1 mg of composition for each cm² of skin surface area (mg/cm²) to 100 mg/cm², preferably from 5 mg/cm² to 10 mg/cm².

The compositions of the present invention as claimed may be applied topically once or more times daily, for example twice, three times or four times daily. The composition is typically applied by spreading it over the skin, generally, only over the affected skin area. The duration of the treatment may be adjusted according to the patient's evolution. Typically, the treatment may be maintained for several weeks, for example 1, 2, 3 or 4 weeks, or several months, for example, 1 to 12 months, or even more, depending on the severity of vitiligo symptoms and the evolution of the disease. The duration of the treatment can be easily adjusted by the skilled practitioner.

Advantageously, the treatment with the composition of the disclosure may be combined with phototherapy, for example, by simple sunlight exposure or by the use of sunbeds, to stimulate melanocyte formation and migration and thus boosting the recovery. Indeed, as shown in Example 4, the composition of the disclosure, combined with sun exposure, lead to almost complete recovery, i.e., complete re-pigmentation of the skin.

The term "phototherapy" or "light therapy" as used herein includes exposure to sunlight or to radiation of specific wavelengths, for example, UV radiation, including UVA and UVB radiation. Therefore, "phototherapy" can be performed by simple exposure to sunlight, or using sunbeds, or using a radiation emitting device, in particular, a UV emitting device.

The duration of the phototherapy is not decisive, and may range, for example, from 10 minutes to 2 hours daily, or on alternate days, or twice or three times weekly, among other suitable options.

Vitiligo can affect any area of skin, so the composition may be applied to any area of affected (depigmented) skin. The areas of skin most commonly affected by vitiligo and, therefore, more in need to be treated, are hands and face, around body openings (the eyes, nostrils, mouth, umbilicus and genital regions), and within body folds such as the underarms and groin.

In one embodiment, the use of the composition of the invention for treating vitiligo as claimed may be combined with the use of antioxidants, in particular, when the therapy is combined with phototherapy, in order to reduce the possible negative effects of the radiation exposure.

The antioxidants are typically for oral use. Suitable antioxidants are, among others, vitamin E, vitamin C, carotenoids (such as lycopene), beta-carotene, alpha-carotene, green tea extract, zinc, selenium, *Polypodium leucotomos* extract, among others, or combinations thereof.

They can be formulated as a dietary oral supplement, typically as tablets, powders or capsules, for example, using standard excipients and preparation process, known in the art.

In one embodiment, the invention as claimed relates to a kit comprising the composition of the invention and a dietary supplement comprising an antioxidant.

In another embodiment, the invention as claimed relates to a kit comprising the composition of the invention and a dietary supplement comprising an antioxidant for use in the prevention and/or treatment of vitiligo.

### Examples

### Example 1 MIA inhibitor peptide

The MIA inhibitor of SEQ ID NO: 40 was used for preparing the compositions of Example 2.

### Preparative example 1: encapsulated MIA inhibitor peptide

Targeted microcapsules of the MIA inhibitor peptide were prepared as bilayered microcapsules wherein the inner polymer was (D,L-lactide-co-glycolide) (PLGA) and the outer polymer was polyvinyl alcohol (PVA), said capsules had attached to the surface the melanocortin 1 (MC1) receptor agonist peptide Palmitoyl-Ser-Tyr-Ser-Nle-Glu-His-DPhe-Arg-Trp-Gly-Lys-DPro-Val-NH-(CH₂)₃-(OCH₂CH₂)₂-CH₂-NH₂ (SEQ ID NO: 50) which was coupled to the capsule by means of an amide bond between the amino terminal group of the peptide and the carboxylic groups available on the surface of the capsule from PLGA. Said microcapsules were prepared using a method analogous to that disclosed in Example 1 of the international patent application WO-A-2015/075116.

The proportion of the MIA inhibitor peptide in the obtained capsules was about 35 wt%, referred to the total weight of the capsules.

Those targeted capsules were incorporated into the composition of the invention as claimed (see Example 2) in the form of an aqueous solution comprising about 0.1 wt% of the capsules (the solution also comprised xanthan gum, phenoxyethanol, caprylyl glycol, glycerin, glyceryl caprylate and phenylpropanol).

### Preparative example 2: gold-anti-MIA peptide conjugate

The MIA inhibitor peptide (SEQ ID NO: 40) was conjugated with gold nanoparticles following a procedure analogous to that disclosed in Examples 1 and 2 of the international patent application WO-A-2019/185696, by first preparing the gold nanoparticles by reducing a gold salt (HAuCl₄) with sodium citrate, and then adding the MIA inhibitor peptide.

The proportion of the MIA inhibitor peptide in the gold conjugate was about 25 wt%, referred to the total weight of the gold-MIA inhibitor conjugate.

This conjugate was incorporated into the composition of the invention as claimed (see Example 2) in the form of an aqueous dispersion comprising about 0.5 wt% of the conjugate (the dispersion also comprised xanthan gum and phenoxyethanol).

### Example 2 Preparation of a composition according to the invention as claimed

A composition according to the present invention as claimed was prepared using the components listed in the following table:

| **Ingredients** | | **Weight%** | | | |
|---|---|---|---|---|---|
| | | **A** | **B** | **C** | **D** |
| A1 | Emulium^{®} Mellifera MB (polyglyceryl-6 distearate, jojoba esters, polyglyceryl-3 beeswax and cetyl alcohol) | 5.0 | 5.0 | 5.0 | 5.0 |
| A2 | Isohexadecane | 2.0 | 2.0 | 2.0 | 2.0 |
| A3 | Isopropyl isostearate | 1.0 | 1.0 | 1.0 | 1.0 |
| A4 | Isononyl Isononanoate | 2.0 | 2.0 | 2.0 | 2.0 |
| A5 | Tocopheryl acetate | 0.2 | 0.2 | 0.2 | 0.2 |
| B1 | Deionized water | 63.9 | 71.9 | 68.9 | 63.9 |
| B2 | Potassium cetyl phosphate | 0.3 | 0.3 | 0.3 | 0.3 |
| B3 | Anhydrous betaine extracted from sugar beet | 2.0 | 2.0 | 2.0 | 2.0 |
| B4 | Preservatives (methylpropanediol, caprylyl glycol and phenylpropanol) | 2.0 | 2.0 | 2.0 | 2.0 |
| B5 | Phenoxyethanol | 0.5 | 0.5 | 0.5 | 0.5 |
| B6 | Disodium EDTA | 0.1 | 0.1 | 0.1 | 0.1 |
| B7 | Glycerin | 2.0 | 2.0 | 2.0 | 2.0 |
| B8 | Xanthan gum | 0.2 | 0.2 | 0.2 | 0.2 |
| C1 | SEPIPLUS^{™} 400 (Polyacrylate-13, Polyisobutene and Polysorbate 20) | 0.5 | 0.5 | 0.5 | 0.5 |
| C2 | Cyclopentasiloxane | 2.0 | 2.0 | 2.0 | 2.0 |
| C3 | Dimethicone | 1.0 | 1.0 | 1.0 | 1.0 |
| D1 | Snail secretion | 5.0 | 5.0 | 5.0 | 5.0 |
| D2a | Anti-MIA peptide encapsulated 0.1 wt% | 10.0 | - | - | - |
| D2b | Anti-MIA peptide-gold conjugate 1 wt% | - | 2.0 | - | - |
| D2c | Anti-MIA peptide 0.1 wt% | - | - | 5.0 | 10.0 |
| D3 | Perfume | 0.3 | 0.3 | 0.3 | 0.3 |
| | Total | 100.0 | 100.0 | 100.0 | 100.0 |

The MIA inhibitor ("anti-MIA") peptide of SEQ ID NO: 40 was used for preparing the compositions. As disclosed in Example 1, it was added either:
- enclosed within melanocyte-targeted capsules (component D2a)
- as a conjugate with gold nanoparticles (component D2b)
- as free peptide (component D2c)

Components D2a and D2b were added as a solution/dispersion, as disclosed in Example 1. Component D2c (free MIA-inhibitor peptide) was added as an aqueous solution (0.1 wt%).

Therefore, the proportion of the MIA inhibitor peptide in the compositions A, B, C and D was about 0.0035% (35 ppm), 0.0025% (25 ppm), 0.005% (50 ppm) and 0.01% (100 ppm), respectively.

For preparing the composition, first components A1-A6 were heated to about 70° C to 75 °C and thoroughly mixed to obtain a first liquid phase ("phase A"). Components B1-B8 were separately mixed to form a solution, which was heated to about 70° C to 75° C. Phase A was added to this solution and was emulsified in a high-shear Ultra-Turrax mixer at 8000 rpm for about 3 minutes and the emulsion was then stirred in a blade mixer at 300 rpm for about 15 minutes. The components C1-C3 were added at 60 °C, the mixture was allowed to cool down to room temperature and components D1, one of D2a/D2b/D2c and D3 were finally added. The final pH of the composition was checked to be in the range 5.5-6.5 (or otherwise adjusted with 10% citric acid or 10% NaOH).

### Example 3 Comparative study to assess the efficacy of the combination MIA inhibitor peptide - snail secretion

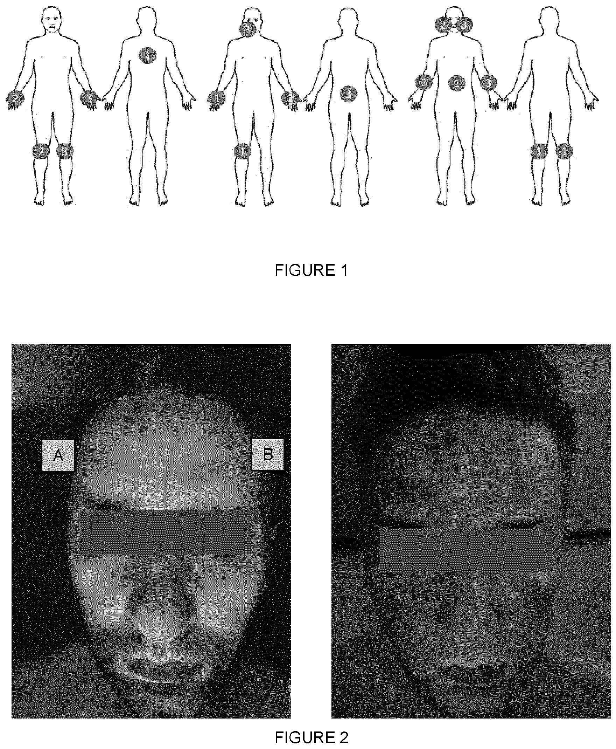

In the assays performed, up to three compositions were comparatively tested in patients suffering from vitiligo. For obtaining reliable comparative data, all three compositions were tested in each patient, by using them at different depigmented areas. Figure 1 shows some examples of randomized treated sites, for example for three hypothetical tested compositions (1, 2 and 3).

For assessing the effect of combining the MIA inhibitor peptide and the snail secretion, the following compositions were compared:

| **Active ingredients** | **Weight%** | | |
|---|---|---|---|
| | **Comparative-I** | **Comparative-II** | **C** |
| Snail secretion | 5 | - | 5 |
| Anti-MIA peptide 0.1 wt% | - | 5 | 5 |

Comparative-I and Comparative-II compositions were prepared using the same excipients A1-A5, B1-B8, C1-C3 and D3 as disclosed in Example 1, in the same amounts, and adjusting the amount of water. Comparative-I formulation comprised 5% of snail secretion (D1) but no MIA inhibitor, and Comparative-II formulation comprised 5% of MIA-inhibitor peptide 0.1% solution (i.e. 50 ppm) but no snail secretion. Formula C comprised both 5% of snail secretion and 50 ppm of the MIA inhibitor peptide. The preparation of the comparative formulas was analogous as disclosed in Example 2.

In the trial, 3 subjects suffering from vitiligo (2 females and 1 male) were treated with the three formulations. Different anatomic sites were treated, including face, chest, armpit, arm, elbow, forearm, wrist, hand, belly, back, buttock, thighs, knee and leg.

The treatment consisted in applying the cream over the treated area twice a day for a period of 3 months.

After the treatment was completed, the efficacy was evaluated by assessing the percentage of re-pigmentation of the treated areas, using a 0-10 scale, wherein 0 means no effect and 10 means 100% of re-pigmentation of the affected area.

The results are shown in the following table:

| **Composition** | **Efficacy** |
|---|---|
| Comparative-1 | 0 |
| Comparative-2 | 2 |
| C | 6 |

Thus, the composition comprising snail secretion alone did not provide any improvement. The composition comprising the MIA inhibitor alone provided only moderate re-pigmentation (of about 20%) of the vitiliginous patches. The composition of the invention as claimed (composition C) comprising both snail secretion and MIA inhibitor provided the best results with almost 60% re-pigmentation of the affected skin areas.

### Example 4 Clinical study to assess the efficacy of the composition as claimed

A prospective clinical study was performed to assess the effectiveness of Formulations A, B and C (Example2).

11 patients (7 females and 4 males, aged 18 to 60) suffering from non-segmental vitiligo were enrolled. 16 different anatomic sites were treated, namely, around the eyes, around the mouth, neck, chest, armpit, arm, elbow, forearm, wrist, hand, belly, back, buttock, thighs, knee and leg.

The three formulations were tested in all the patients, by using them at different depigmented areas. The treated sites were randomized, for example, as shown in Figure 1.

The treatment consisted in applying the cream over the treated area twice a day for a period of 5 months. During the treatment, the patients were requested to get some sun exposure or to use sunbeds. The patients were also requested to take an oral anti-oxidant composition (comprising *Polypodium leucotomos* extract, green tea extract and vitamin E) in order to neutralize any possible adverse effect derived from sun exposure.

9 patients (i.e 82% of the patients) showed at least partial re-pigmentation with all the three treatments assayed, while 2 of the patients were non-respondent. Most of respondents showed outstanding re-pigmentation of the vitiliginous depigmented areas, with a degree of re-pigmentation of 90% or higher.

No significant differences were found between the three tested compositions (A, B and C).

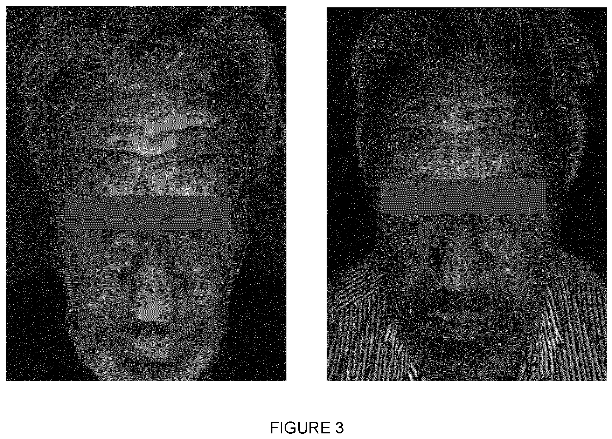

For Example, Figure 2 shows the face of one patient, before and after the treatment, who was treated with composition A on the left side and composition B on the right side. Figure 3 shows the face of another patient, before and after the treatment, who was treated with composition C.

## Claims

1. A pharmaceutical composition comprising:
(a) a melanoma inhibitory activity protein (MIA) inhibitor peptide, which has sequence SEQ ID NO: 40, or is a derivative thereof, wherein derivative means that the peptide is acetylated at the N-terminus and/or amidated at the C-terminus;
(b) snail secretion; and
(c) at least one pharmaceutically acceptable excipient.

2. The pharmaceutical composition according to claim 1, **characterized in that** the C-terminal amidation consists in the formation of terminal amido groups of formula - CONH₂, -CONHR or -CONR₂, wherein R is a C₁-C₄ alkyl group.

3. The pharmaceutical composition according to claims 1 or 2, **characterized in that** the MIA inhibitor peptide is encapsulated or is conjugated with a carrier nanoparticle.

4. The pharmaceutical composition according to claim 3, **characterized in that** the MIA inhibitor peptide is encapsulated within a microcapsule or nanocapsule which has attached on the outer surface a specific peptide which is a melanocortin 1 (MC1) receptor agonist.

5. The pharmaceutical composition according to claim 3, **characterized in that** the MIA inhibitor peptide is conjugated with gold nanoparticles.

6. The pharmaceutical composition according to any one of claims 1 to 5, **characterized in that** the amount of the MIA inhibitor peptide in the composition is comprised between 0.0001% and 1%, expressed as weight of the MIA inhibitor relative to the total weight of the composition.

7. The pharmaceutical composition according to claim 6, **characterized in that** the amount of the MIA inhibitor peptide in the composition is comprised between 0.0005% and 0.1%, expressed as weight of the MIA inhibitor relative to the total weight of the composition.

8. The pharmaceutical composition according to any one of claims 1 to 7, **characterized in that** the amount of snail secretion in the composition is comprised between 0.5% and 15%, expressed as weight of the snail secretion relative to the total weight of the composition.

9. The pharmaceutical composition according to any one of claims 1 to 8, **characterized in that** it is for topical use.

10. The pharmaceutical composition according to claim 9, **characterized in that** it is selected from a cream and a gel.

11. A kit comprising the pharmaceutical composition according to any one of claims 1 to 10 and a dietary supplement comprising an antioxidant.

12. The pharmaceutical composition according to any one of claims 1 to 10 or the kit according to claim 11 for use in the prevention and/or treatment of vitiligo.

## Patentansprüche

1. Pharmazeutische Zusammensetzung, umfassend:
(a) ein melanominhibierendes Aktivitätsprotein (MIA)-Inhibitorpeptid, das die Sequenz SEQ ID NO: 40 aufweist, oder ein Derivat davon ist, wobei Derivat bedeutet, dass das Peptid am N-Terminus acetyliert und/oder am C-Terminus amidiert ist;
(b) Schneckensekret; und
(c) mindestens einen pharmazeutisch akzeptablen Hilfsstoff.

2. Pharmazeutische Zusammensetzung nach Anspruch 1, **dadurch gekennzeichnet, dass** die C-terminale Amidierung in der Bildung von terminalen Amidogruppen der Formel -CONH₂, -CONHR oder -CONR₂ besteht, wobei R eine C₁-C₄-Alkylgruppe ist.

3. Pharmazeutische Zusammensetzung nach Anspruch 1 oder 2, **dadurch gekennzeichnet, dass** das MIA-Inhibitorpeptid eingekapselt ist oder mit einem Träger-Nanopartikel konjugiert ist.

4. Pharmazeutische Zusammensetzung nach Anspruch 3, **dadurch gekennzeichnet, dass** das MIA-Inhibitorpeptid in einer Mikrokapsel oder Nanokapsel eingekapselt ist, an deren Außenfläche ein spezifisches Peptid gebunden ist, das ein Melanocortin-1(MC1)-Rezeptor-Agonist ist.

5. Pharmazeutische Zusammensetzung nach Anspruch 3, **dadurch gekennzeichnet, dass** das MIA-Inhibitorpeptid mit Gold-Nanopartikeln konjugiert ist.

6. Pharmazeutische Zusammensetzung nach einem der Ansprüche 1 bis 5, **dadurch gekennzeichnet, dass** die Menge des MIA-Inhibitorpeptids in der Zusammensetzung zwischen 0,0001 % und 1 % liegt, ausgedrückt als Gewicht des MIA-Inhibitors bezogen auf das Gesamtgewicht der Zusammensetzung.

7. Pharmazeutische Zusammensetzung nach Anspruch 6, **dadurch gekennzeichnet, dass** die Menge des MIA-Inhibitorpeptids in der Zusammensetzung zwischen 0,0005 % und 0,1 % liegt, ausgedrückt als Gewicht des MIA-Inhibitors bezogen auf das Gesamtgewicht der Zusammensetzung.

8. Pharmazeutische Zusammensetzung nach einem der Ansprüche 1 bis 7, **dadurch gekennzeichnet, dass** die Menge des Schneckensekrets in der Zusammensetzung zwischen 0,5 % und 15 % liegt, ausgedrückt als Gewicht des Schneckensekrets bezogen auf das Gesamtgewicht der Zusammensetzung.

9. Pharmazeutische Zusammensetzung nach einem der Ansprüche 1 bis 8, **dadurch gekennzeichnet, dass** sie zur topischen Anwendung bestimmt ist.

10. Pharmazeutische Zusammensetzung nach Anspruch 9, **dadurch gekennzeichnet, dass** sie aus einer Creme und einem Gel ausgewählt ist.

11. Kit, umfassend die pharmazeutische Zusammensetzung nach einem der Ansprüche 1 bis 10 und ein Nahrungsergänzungsmittel, das ein Antioxidans umfasst.

12. Pharmazeutische Zusammensetzung nach einem der Ansprüche 1 bis 10 oder Kit nach Anspruch 11 zur Verwendung bei der Prävention und/oder Behandlung von Vitiligo.

## Revendications

1. Composition pharmaceutique comprenant :
(a) un peptide inhibiteur de la protéine d'activité inhibitrice du mélanome (MIA), qui a la séquence SEQ ID NO : 40, ou est un dérivé de celle-ci, dans laquelle le dérivé signifie que le peptide est acétylé à l'extrémité N-terminale et/ou amidé à l'extrémité C-terminale ;
(b) une sécrétion d'escargot ; et
(c) au moins un excipient pharmaceutiquement acceptable.

2. Composition pharmaceutique selon la revendication 1, **caractérisé en ce que** l'amidation de l'extrémité C-terminale consiste en la formation de groupes amido terminaux de formule -CONH₂, -CONHR ou -CONR₂, dans laquelle R est un groupe alkyle en C₁-C₄.

3. Composition pharmaceutique selon la revendication 1 ou 2, **caractérisée en ce que** le peptide inhibiteur de MIA est encapsulé ou est conjugué à une nanoparticule porteuse.

4. Composition pharmaceutique selon la revendication 3, **caractérisée en ce que** le peptide inhibiteur de MIA est encapsulé dans une microcapsule ou une nanocapsule à la surface extérieure de laquelle est fixé un peptide spécifique qui est un agoniste du récepteur de la mélanocortine 1 (MC1).

5. Composition pharmaceutique selon la revendication 3, **caractérisée en ce que** le peptide inhibiteur de MIA est conjugué à des nanoparticules d'or.

6. Composition pharmaceutique selon l'une quelconque des revendications 1 à 5, **caractérisée en ce que** la quantité du peptide inhibiteur de MIA dans la composition est comprise entre 0,0001 % et 1 %, exprimée en poids de l'inhibiteur de MIA par rapport au poids total de la composition.

7. Composition pharmaceutique selon la revendication 6, **caractérisée en ce que** la quantité du peptide inhibiteur de MIA dans la composition est comprise entre 0,0005 % et 0,1 %, exprimée en poids de l'inhibiteur de MIA par rapport au poids total de la composition.

8. Composition pharmaceutique selon l'une quelconque des revendications 1 à 7, **caractérisée en ce que** la quantité de sécrétion d'escargot dans la composition est comprise entre 0,5 % et 15 %, exprimée en poids de la sécrétion d'escargot par rapport au poids total de la composition.

9. Composition pharmaceutique selon l'une quelconque des revendications 1 à 8, **caractérisée en ce qu'**elle est destinée à un usage topique.

10. Composition pharmaceutique selon la revendication 9, **caractérisée en ce qu'**elle est choisie parmi une crème et un gel.

11. Kit comprenant la composition pharmaceutique selon l'une quelconque des revendications 1 à 10 et un complément alimentaire comprenant un antioxydant.

12. Composition pharmaceutique selon l'une quelconque des revendications 1 à 10 ou kit selon la revendication 11 pour une utilisation dans la prévention et/ou le traitement du vitiligo.
